# EUROPEAN PATENT APPLICATION

(11) **EP 4 454 584 A1**
(43) Date of publication of application: **30.10.2024**
(21) Application number: 22921538.9
(22) Date of filing: 10.10.2022
(51) Int. Cl.: A61B 18/14, A61B 18/12

(54) **SAFE HIGH-FREQUENCY ELECTROTOME SURGICAL INSTRUMENT**

(30) Priority: 24.01.2022 CN 202210081330
(71) Applicant: Innolcon Medical Technology (Suzhou) Co., Ltd., Jiangsu 215000 (CN)
(72) Inventor: WANG, Zhiping, Suzhou, Jiangsu 215000 (CN); YUAN, Xiaohe, Suzhou, Jiangsu 215000 (CN); WEI, Dalun, Suzhou, Jiangsu 215000 (CN); LUO, Wei, Suzhou, Jiangsu 215000 (CN)
(74) Representative: Doherty, William
(86) International application number: PCT/CN2022/124306
(87) International publication number: WO 2023/138111

(57) **Abstract**

A safe high-frequency electrotome surgical instrument, including a housing (1), a shaft rod member, a jaw assembly, a control wrench (2), a blade (3), and a control button (4). The control wrench (2) controls opening and closing of the jaw assembly through a jaw control mechanism. The control button (4) drives the blade (3) to move for cutting through a blade control mechanism. The moving direction of the control wrench (2) is consistent with that of the control button (4), and a locking mechanism is disposed between the control wrench (2) and the control button (4). The locking mechanism is composed of a locking lug boss (5) and an inverted buckle (6) capable of being clamped with the locking lug boss (5). The locking lug boss (5) is disposed on the control wrench (2), and the inverted buckle (6) is disposed on the control button (4) or on a triggering connecting rod (402). In a first state, the jaw assembly is opened, and the locking lug boss (5) is clamped with the inverted buckle (6), such that the control button (4) is not movable. In a second state, the jaw assembly is closed, and the locking lug boss (5) is separated from the inverted buckle (6), such that the control button (4) is movable to drive the blade (3) for cutting. A locking structure and a turnback mechanism are disposed to ensure that the blade (3) performs telescopic cutting when the jaw assembly is closed, thereby ensuring operational safety.

## Description

### FIELD OF THE INVENTION

The present invention relates to the technical field of surgical instruments, and in particular, to a safe high-frequency electrotome surgical instrument.

### BACKGROUND OF THE INVENTION

There is a jaw and a blade at the operating end of a high-frequency electrotome surgical instrument, so that the high-frequency electrotome surgical instrument has multiple functions, such as clamping tissue, coagulating tissue with high-frequency electrical energy, and cutting tissue with a blade. At least a jaw control mechanism for controlling the jaw to be opened and closed to clamp tissue, and a blade control mechanism for controlling the blade to telescopically cut tissue need to be disposed inside the high-frequency electrotome surgical instrument. Usually, the jaw control mechanism and the blade control mechanism are disposed independently, thereby facilitating step-by-step operations of the jaw and the blade.

A typical operation process of the high-frequency electrotome surgical instrument is to first control the jaw to be closed to clamp tissue, and then cut with the blade. For such operation process, it is needed to ensure the close of the jaw during a cutting process of the blade. In other words, the jaw control mechanism needs to be controlled continuously. The control end of existing jaw control mechanism is mostly a handle, which has high requirements on holding stability of an operator. For some existing high-frequency electrotome surgical instruments, locking mechanisms may be disposed for locking the jaw control mechanisms. However, the locking mechanisms can easily affect operational flexibility of the jaw control mechanism, resulting in locking failures or resetting failures after locking. As a result, the opening and closing of the jaw are not smooth, resulting in inconvenience in operations.

On the other hand, because the blade control mechanism and the jaw control mechanism operate independently, and to facilitate a handheld operation, the blade control mechanism and the jaw control mechanism are usually disposed on the handheld handle of the high-frequency electrotome surgical instrument. In practical operations, accidental triggering may be caused because the blade control mechanism and the jaw control mechanism are disposed too close to each other, especially when the blade is accidentally triggered before the jaw is controlled to grip tissue. As a result, tissue is accidentally cut while gripping effects are affected. This brings high difficulties and great inconvenience to the operation of the high-frequency electrotome surgical instrument. At the same time, there are significant safety hazards.

### SUMMARY OF THE INVENTION

An objective of the present invention is to overcome shortcomings of existing technologies, and provide a safe high-frequency electrotome surgical instrument and a surgical instrument.

The objective of the present invention is achieved through the following technical solutions.

A safe high-frequency electrotome surgical instrument, including a housing, a shaft rod member, a jaw assembly, a control wrench, a blade, and a control button, where the control wrench controls opening and closing of the jaw assembly through a jaw control mechanism; the control button drives the blade to move for cutting through a blade control mechanism; the moving direction of the control wrench is consistent with that of the control button, and a locking mechanism is disposed between the control wrench and the control button; the locking mechanism is composed of a locking lug boss and an inverted buckle capable of being clamped with the lug boss; the locking lug boss is disposed on the control wrench; the inverted buckle is disposed on the control button or is disposed on a triggering connecting rod of the blade control mechanism; in a first state, the jaw assembly is opened, and the locking lug boss is clamped with the inverted buckle, such that the control button is not movable; in a second state, the jaw assembly is closed, and the locking lug boss is separated from the inverted buckle, such that the control button is movable to drive the blade for cutting; and after the control wrench is turned back, the locking lug boss is re-clamped with the inverted buckle.

Preferably, the shaft rod member extends forward from the front end of the housing and defines a longitudinal axis; the shaft rod member includes an inner sleeve and an outer sleeve; the jaw assembly is disposed at the front end of the shaft rod member; and the blade is disposed inside the inner sleeve.

Preferably, the jaw assembly includes a first jaw disposed at the front end of the inner sleeve and a second jaw disposed at the front end of the outer sleeve; the first jaw is hingedly connected to the second jaw through a first hinge pin; guide slots are disposed on side portions of the tail ends of both the first jaw and the second jaw; the front end of the inner sleeve is connected to the first jaw through a second hinge pin; the two ends of the second hinge pin are movably clamped in the guide slots; the guide slot is enclosed by a baffle; and the inner sleeve drives the first jaw and the second jaw to be opened and closed by moving longitudinally.

Preferably, the jaw control mechanism includes a closing connecting rod, a push block, and a jaw spring; the push block is sleeved on the inner sleeve, and abuts against the jaw spring that is sleeved at the tail end of the inner sleeve; and the control wrench is connected to the push block through the closing connecting rod, and drives the push block and the inner sleeve to move longitudinally and synchronously.

Preferably, a push-block guide slot defining the moving direction of the push block is disposed on an inner wall of the housing; and the push block is slidably clamped in the push-block guide slot.

Preferably, a jaw reset spring abuts against the tail end of the push block; and the jaw reset spring is clamped in a limiting slot on the inner wall of the housing.

Preferably, a wrench locking mechanism is disposed between the bottom portion of the control wrench and the housing; the wrench locking mechanism includes a locking block located on the side portion of the control wrench and a locking rod located inside the housing; a clamping slot containing a sliding guide surface is formed on the surface of the locking block; a limiting slot is disposed inside the housing; the locking rod moves elastically along the limiting slot, and the tail end of the locking rod is pivotally connected to the housing; and there is a protruding portion at the front end of the locking rod, so that the locking block is enabled to drive, through movement relative to the locking rod, the protruding portion to slide along the clamping slot.

Preferably, a Z-shaped/concave guide surface is formed on the surface of the clamping slot; an inward bending portion is formed on the middle portion of the clamping slot; when the protruding portion slides along the guide surface to the inward bending portion, the locking block and the locking rod are locked; and when the protruding portion slides out of the inward bending portion, the locking block and the locking rod are unlocked.

Preferably, elastic members are abutted on the upper and lower sides of the side wall of the housing along the pivot direction of the locking rod.

Preferably, two conductive rings are respectively mounted outside the outer sleeve; and the two conductive rings are electrically connected to the first jaw and the second jaw through conducting wires, respectively.

Preferably, conductive sheets slidably abut against the two conductive rings, respectively; the two conductive sheets are fixedly disposed on the inner wall of the housing, and are connected to a power supply through conducting wires, respectively; a knob is disposed at the front end of the housing; and the knob is fixedly connected to the outer sleeve and drives the outer sleeve to rotate synchronously.

Preferably, a blade holder of the blade is inserted into the inner sleeve; and the blade is movably clamped in a movable slot between the first jaw and the second jaw

Preferably, the blade control mechanism includes the triggering connecting rod, a blade feeding clip, and a blade reset spring; the blade feeding clip is connected to the blade holder; the blade reset spring is sleeved on the inner sleeve, and abuts against the blade feeding clip; the control button is connected to the blade feeding clip through the triggering connecting rod; a button guide slot is disposed inside the housing; and the control button slides along the button guide slot and controls the blade feeding clip to move along the inner sleeve, to drive the blade to move.

Preferably, a convex post is disposed on the control wrench, the convex post is located above the locking lug boss; a concave portion matching the convex post is disposed on the triggering connecting rod; the convex post and the concave portion of the triggering connecting rod form an auxiliary turnback mechanism; and when the control wrench is turned back, the convex post abuts against the concave portion of the triggering connecting rod, and drives the triggering connecting rod to be synchronously turned back, where the control button is synchronously turned back with the triggering connecting rod.

Beneficial effects of the present invention are mainly as below
1. A locking structure is disposed, so that the control wrench plays a limiting role on the control button. When the jaw assembly is opened, the locking lug boss is clamped with the inverted buckle, so that the control button is not movable, that is, the blade cannot extend out. When the jaw assembly is closed, the locking lug boss moves backward and is separated from the inverted buckle, so that the control button is movable to drive the blade to extend out, thereby ensuring that the blade only moves for cutting after tissue is clamped by the jaw, thereby ensuring operational safety, and preventing the blade from being accidentally triggered to move for cutting.
2. The wrench locking mechanism is disposed between the bottom portion of the control wrench and the housing, to automatically lock the movement of the wrench, thereby reducing control pressure on hands of an operator and reducing operational difficulty. Due to a Z-shaped clamping slot, the position of the control wrench relative to the housing may be locked or unlocked by repeatedly moving the control wrench, with high operational smoothness.
3. The conductive sheet is disposed to cooperate with the conductive ring, so that the outer sleeve can rotate freely while current conduction is ensured.
4. The convex post is disposed to cooperate with the triggering connecting rod to form the auxiliary turnback mechanism, which cooperates with the jaw reset spring to have an auxiliary turnback function on the control button. When the control wrench is turned back, the convex post may drive the triggering connecting rod to be turned back synchronously, so as to drive the control button to be turned back synchronously.

### BRIEF DESCRIPTION OF THE DRAWINGS

Technical solutions of the present invention are further described below with reference to the accompanying drawings.
FIG. 1 is a schematic diagram according to an embodiment of the present invention;
FIG. 1-1 is a schematic diagram according to another feasible embodiment of the present invention;
FIG. 2 is a schematic diagram of a jaw assembly according to an embodiment of the present invention;
FIG. 3 is an exploded schematic diagram of a shaft rod member according to an embodiment of the present invention;
FIG. 4 is an exploded schematic diagram of a part of a shaft rod member according to an embodiment of the present invention;
FIG. 5 is a schematic diagram of a part of a structure according to an embodiment of the present invention;
FIG. 6 is a schematic diagram in a first state according to an embodiment of the present invention;
FIG. 7 is a schematic diagram of switching from a first state to a second state according to an embodiment of the present invention;
FIG. 8 is a schematic diagram of switching from a first state to a second state according to an embodiment of the present invention;
FIG. 9 is a schematic diagram in a second state according to an embodiment of the present invention; and
FIG. 10 is an internal schematic diagram according to another embodiment of the present invention.

### DETAILED DESCRIPTION OF THE EMBODIMENTS

The present invention is described in detail below with reference to specific embodiments shown in the accompanying drawings. However, these embodiments are not limited to the present invention, and changes in structure, method, or function that are made by persons of ordinary skills in the art based on these embodiments are all included in the protection scope of the present invention.

In the description of the embodiments, it should be noted that, orientations or positional relationships indicated by terms such as "center", "up", "down", "left", "right", "front", "rear", "vertical", "horizontal", "inside", "outside", etc. are based on the orientations or positional relationships shown in the accompanying drawings, and are only for convenience of description and for simplifying the description, rather than being intended to indicate or imply that a device or an element referred to must have a particular orientation or be constructed and operated in a particular orientation, and therefore should not be construed as a limitation to the present invention. In addition, terms such as "first", "second", and "third" are merely used for description, and cannot be understood as indicating or implying relative importance. Moreover, in the description of the embodiments, taking an operator as reference, a direction close to the operator is a proximal end, and a direction away from the operator is a distal end.

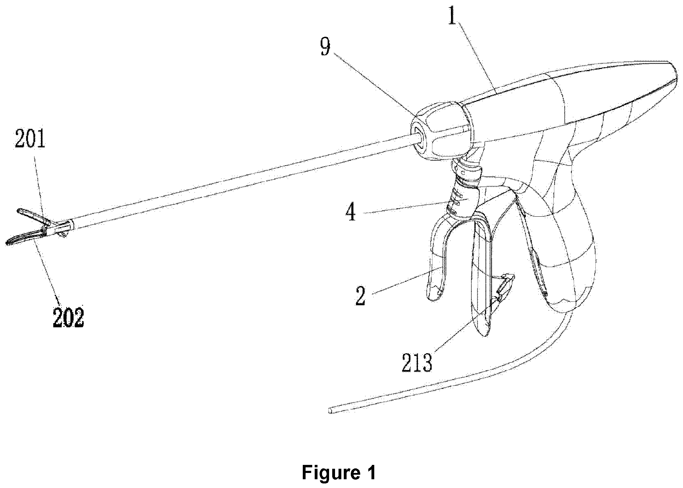

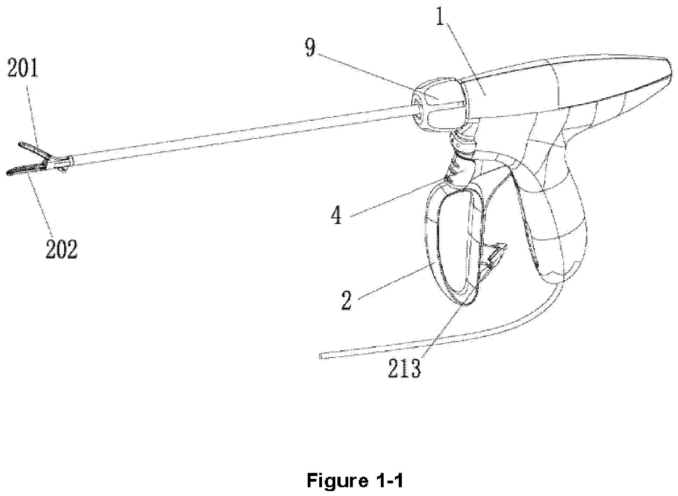

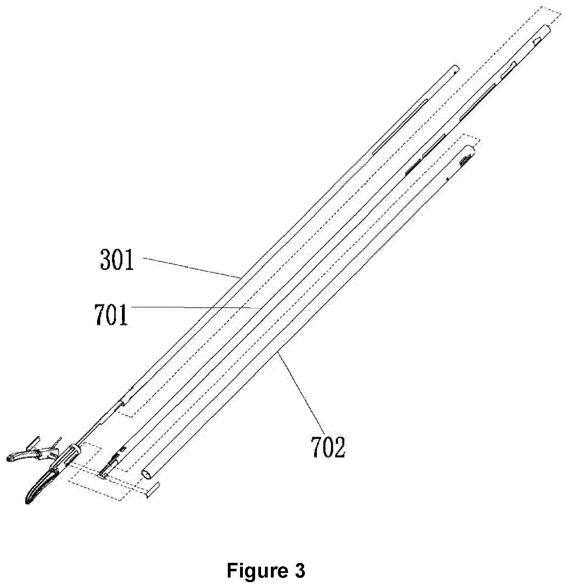

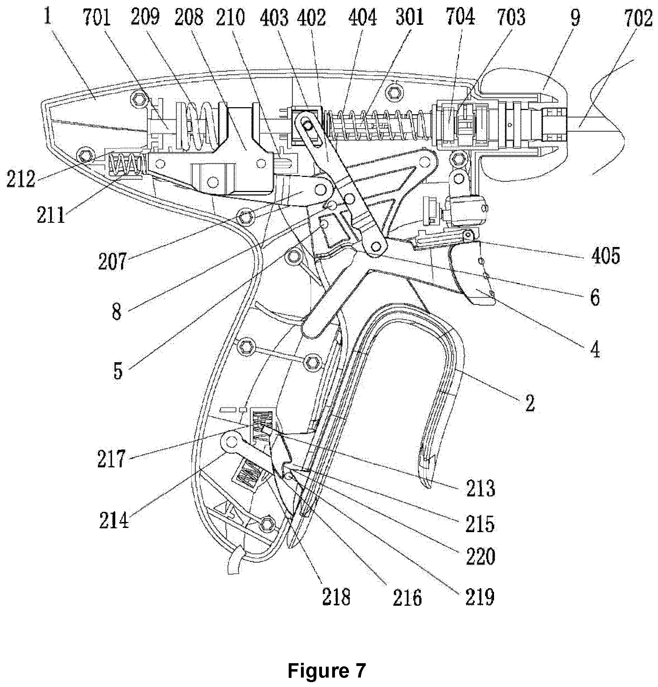

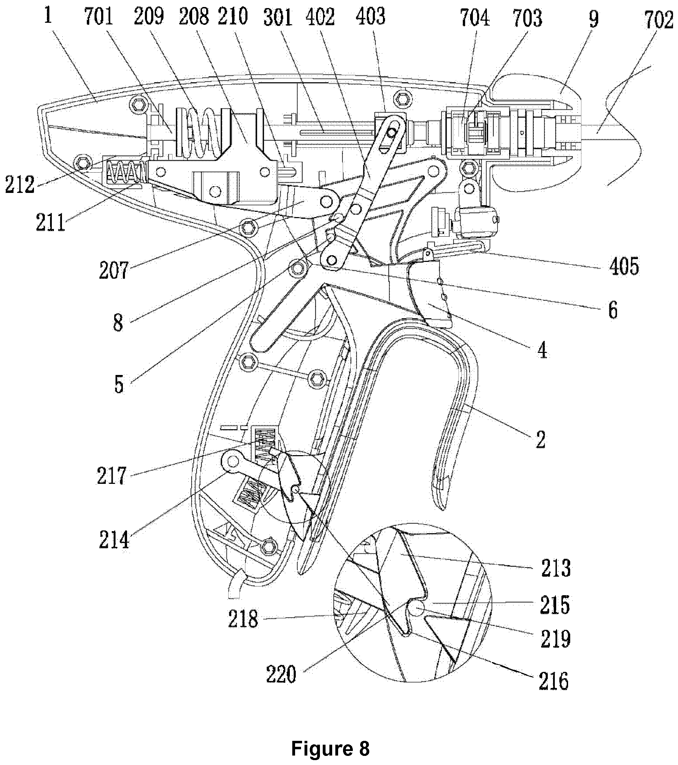

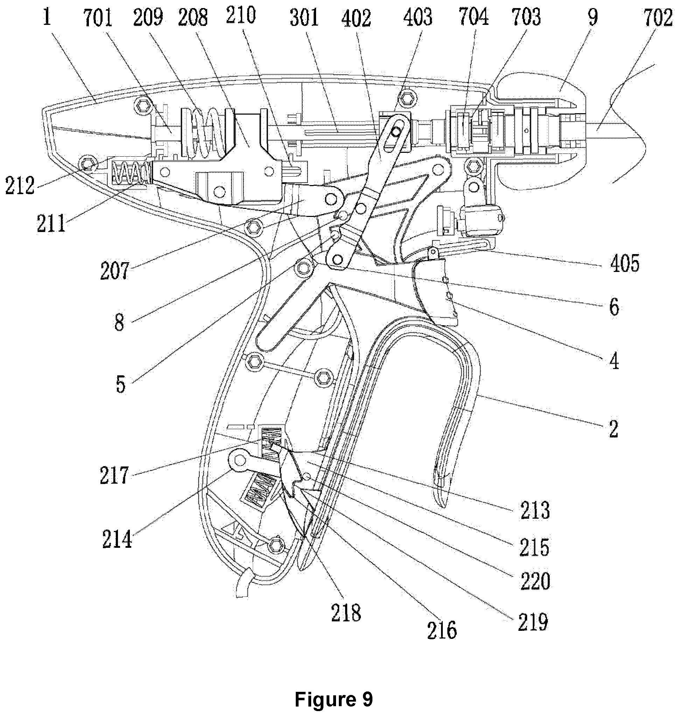

As shown in FIG. 1 to FIG. 9, a safe high-frequency electrotome surgical instrument is disclosed in the present invention, including a housing 1, a shaft rod member, a jaw assembly, a control wrench 2, a blade 3, and a control button 4. The control wrench 2 controls opening and closing of the jaw assembly through a jaw control mechanism. The control button 4 drives the blade 3 to move for cutting through a blade control mechanism. The moving direction of the control wrench 2 is consistent with that of the control button 4, and a locking mechanism is disposed between the control wrench 2 and the control button 4. The locking mechanism is composed of a locking lug boss 5 and an inverted buckle 6 capable of being clamped with the lug boss 5. The locking lug boss 5 is disposed on the control wrench 2. The inverted buckle 6 is disposed on the control button 4 or is disposed on a triggering connecting rod 402 of the blade control mechanism. In a first state, the jaw assembly is opened, and the locking lug boss 5 is clamped with the inverted buckle 6, such that the control button 4 is not movable. In a second state, the jaw assembly is closed, and the locking lug boss 5 is separated from the inverted buckle 6, such that the control button 4 is movable to drive the blade 3 for cutting. After the control wrench 2 is turned back, the locking lug boss 5 is re-clamped with the inverted buckle 6.

According to the present invention, a locking mechanism is disposed between the control wrench 2 and the control button 4, so that the control wrench 2 has a limiting effect on the control button 4. When the jaw assembly is opened, the locking lug boss 5 is clamped with the inverted buckle 6, so that the control button 4 is movable, that is, the blade 3 cannot extend out. When the jaw assembly is closed, the locking lug boss 5 moves backward and is separated from the inverted buckle 6, so that the control button 4 has movable space for moving and driving the blade 3 for telescopic cutting. The disposing of the locking mechanism may ensure that the blade 3 only moves for cutting after a jaw is closed to clamp tissue, thereby ensuring the safety of use, and avoiding accidental cutting caused by the movement of the blade 3 being triggered accidentally.

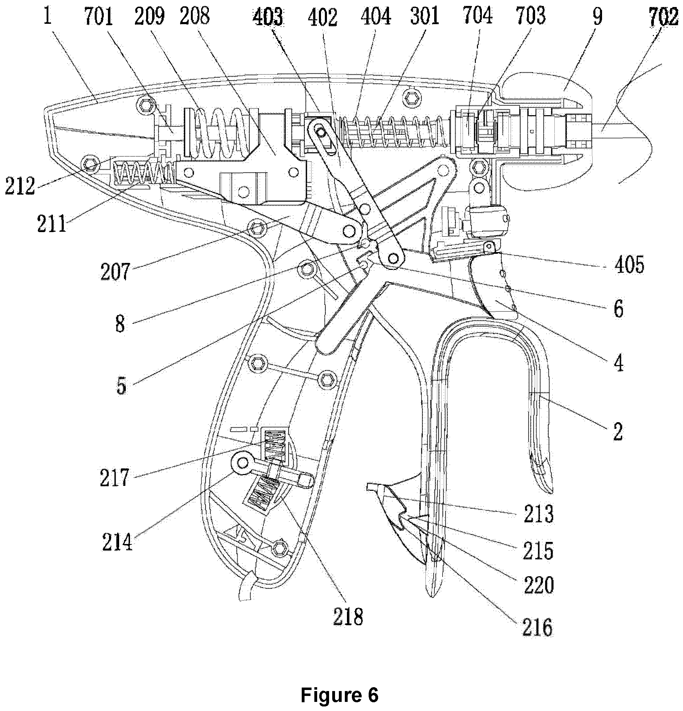

Specifically, as shown in FIG. 6 to FIG. 9, the locking lug boss 5 is disposed on an extension portion of the control wrench 2 located inside the housing 1. The inverted buckle 6 is preferably disposed at the edge of the tail end of the control button 4. The locking lug boss 5 is clamped with the inverted buckle 6 to form a locking function, so as to directly limit the movement of the control button 4. In other feasible embodiments, the inverted buckle 6 may also be disposed at another proper position on the control button 4.

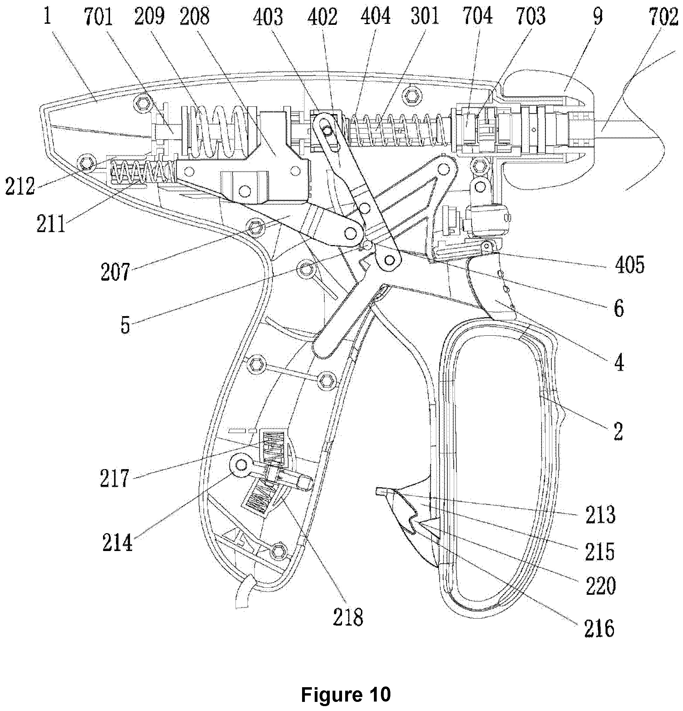

As shown in FIG. 10, in another feasible embodiment, the locking lug boss 5 is not in contact with the control button 4, and the inverted buckle 6 is disposed at any position on a side edge or a side portion of the triggering connecting rod 402 that may ensure its clamping with the locking lug boss 5. The locking lug boss 5 is clamped with the inverted buckle 6, forming a locking function by limiting the movement of the triggering connecting rod 402 to limit the movement of the control button 4indirectly. A working principle is: The triggering connecting rod 402 abuts against the locking lug boss 5, and the torque of the contact pressure on the control wrench 2 is consistent with the turnback direction of the control wrench 2, achieving the purpose of stopping the control button 4.

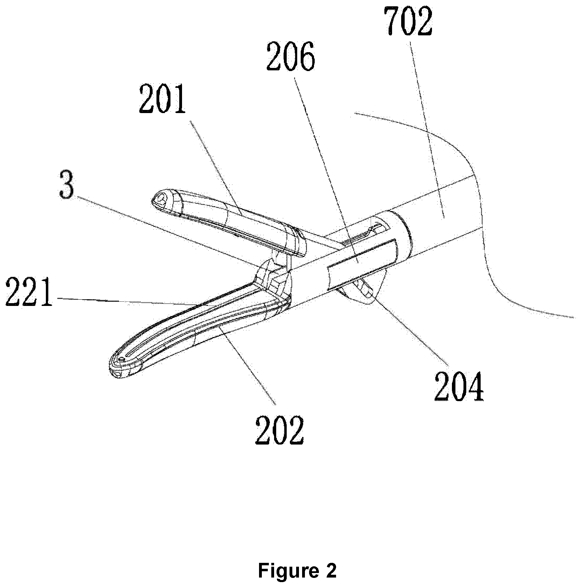

Specifically, as shown in FIG. 2, the shaft rod member extends forward from the front end of the housing 1 and defines the longitudinal axis. The shaft rod member includes an inner sleeve 701 and an outer sleeve 702. The jaw assembly is disposed at the front end of the shaft rod member. The blade 3 is disposed inside the inner sleeve 701.

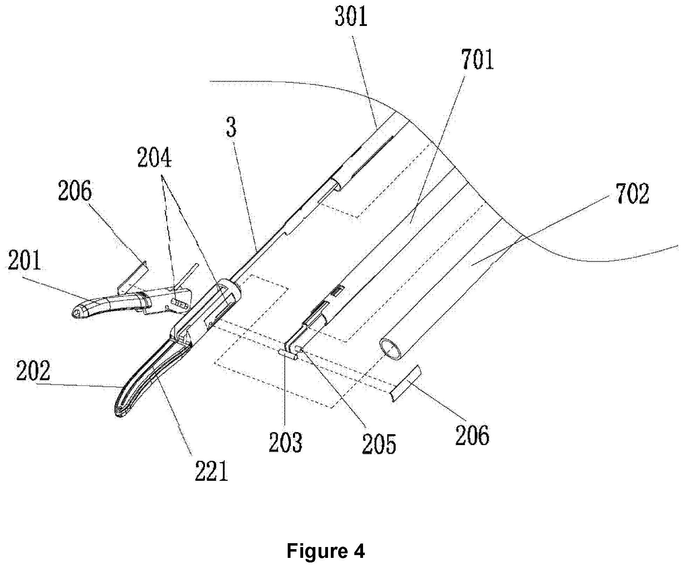

As shown in FIG. 4, in this embodiment, the jaw assembly includes a first jaw 201 disposed at the front end of the inner sleeve 701 and a second jaw 202 disposed at the front end of the outer sleeve 702. The first jaw 201 is hingedly connected to the second jaw 202 through a first hinge pin 203. Guide slots 204 are disposed on side portions of the tail ends of both the first jaw 201 and the second jaw 202. The front end of the inner sleeve 701 is connected to the first jaw 201 through a second hinge pin 205, and the two ends of the second hinge pin 205 are movably clamped in the guide slots 204, the inner sleeve 701 drives the first jaw 201 and the second jaw 202 to be opened and closed by moving longitudinally. In this preferred embodiment, the guide slot 204 is enclosed through a baffle 206, which plays a limiting role on the first hinge pin 203 and the second hinge pin 205. Such structural arrangement makes the first hinge pin 203 and the second hinge pin 205 cylindrical only, facilitating manufacturing and mounting. In other feasible embodiments, the first hinge pin 203 and the second hinge pin 205 may also form an end portion with a diameter greater than their rod body to play a limiting role, or other components with a limiting role may be used. A working principle of the jaw assembly is: When the inner sleeve 701 moves forward, it may drive the second hinge pin 205 to synchronously move forward in the guide slot 204. Because the first jaw 201 is hingedly connected to the second jaw 202 through the first hinge pin 203, the forward movement of the second hinge pin 205 may cause the front end of the first jaw 201 to tilt upward, causing the jaw assembly to be opened. When the inner sleeve 701 moves backward, it may drive the second hinge pin 205 to synchronously move backward in the guide slot 204, causing the front end of the first jaw 201 moves downward until it is in contact with the second jaw 202, causing the jaw assembly to be closed.

Further, as shown in FIG. 6 to FIG. 9, the jaw control mechanism includes a closing connecting rod 207, a push block 208, and a jaw spring 209. One end of the extension portion of the control wrench 2 located inside the housing 1 is pivotally connected to the housing 1, and the other end is pivotally connected to the closing connecting rod 207. The push block 208 is sleeved on the inner sleeve 701, and abuts against the jaw spring 209 sleeved at the tail end of the inner sleeve 701. The control wrench 2 is connected to the push block 208 through the closing connecting rod 207, and drives the push block 208 and the inner sleeve 701 to move longitudinally and synchronously.

To improve smoothness and stability of the movement of the jaw control mechanism, a push-block guide slot 210 that defines the moving direction of the push block 208 is disposed on the inner wall of the housing 1. The push block 208 is slidably clamped in the push-block guide slot 210, to ensure that the moving direction of the push block 208 is consistent with the extension direction of the inner sleeve 701, thereby improving moving smoothness of the inner sleeve 701, and avoiding frictions between the inner sleeve 701 and other components.

Further, a jaw reset spring 211 abuts against the tail end of the push block 208; and the jaw reset spring 211 is clamped in a limiting slot 212 on the inner wall of the housing 1. The disposing of the jaw reset spring 211 increases driving force for resetting the push block 208, thereby ensuring reset of the push block 208.

As shown in FIG. 6 to FIG. 9, to further facilitate operations of the control wrench 2, a wrench locking mechanism is disposed between the bottom portion of the control wrench 2 and the housing 1. The wrench locking mechanism includes a locking block 213 located on the side portion of the control wrench 2 and a locking rod 214 located in the handle portion of the housing 1. The tail end of the locking rod 214 is pivotally connected to the housing 1, and elastic members 217 symmetrically abut against both upper and lower sides of the moving direction of the locking rod 214. The elastic members 217 are preferably springs. In other embodiments, the elastic members 217 may also be of other elastic structures. The elastic members 217 are symmetrically disposed, so as to ensure that the upper and lower sides of the locking rod 214 are balanced in force, thereby ensuring that the locking rod 214 is maintained at a constant position. In this preferred embodiment, the locking rod 214 is maintained in a slightly downward inclined state , so that the front end of the clamping slot 215 is always higher than the front end of the locking rod 214. In this case, when the clamping slot 215 approaches the locking rod 214, the locking rod 214 may be driven to move along the outer contour of the clamping slot 215. At the same time, the locking rod 214 is slidably clamped in an arc-shaped guide slot 218 that is disposed inside the housing 1, and moves arcuately along the arc-shaped guide slot 218. The arc-shaped guide slot 218 plays a limiting role on the movement of the locking rod 214, thereby further improving movement stability of the locking rod 214.

The clamping slot 215 is formed on the surface of the locking block 213. A Z-shaped/concave guide surface 216 is formed on the surface of the clamping slot 215, and an inward bending portion 220 is formed on the middle portion of the clamping slot 215. There is a protruding portion 219 at the front end of the locking rod 214. The locking block 213 drives, through moving relative to the locking rod 214, the protruding portion 219 to slide along the guide surface 216. The inward concave structure of the inward bending portion 220 relative to the locking rod 214 plays a role of locking the protruding portion 219. When the protruding portion 219 slides into the inward bending portion 220 of the guide surface 216, the locking block 213 and the locking rod 214 are locked. When the protruding portion 219 slides out of the inward bending portion 220, the locking block 213 and the locking rod 214 are unlocked.

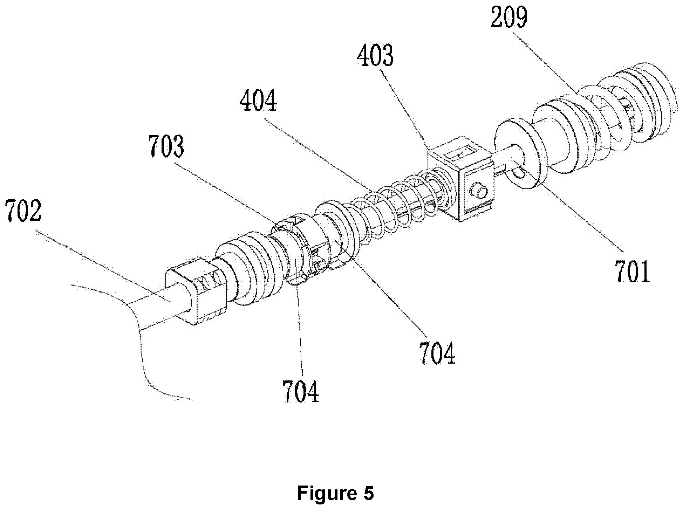

As shown in FIG. 5, two conductive rings 703 are respectively mounted outside the outer sleeve 702. The two conductive rings 703 are electrically connected to the first jaw 201 and the second jaw 202 through conducting wires, respectively.

Further, conductive sheets 704 slidably abut against the two conductive rings 703, respectively. The two conductive sheets 704 are fixedly disposed on the inner wall of the housing 1, and are connected to a power supply through conducting wires, respectively. A knob 9 is disposed at the front end of the housing 1. The knob 9 is fixedly connected to the outer sleeve 702 and drives the outer sleeve 702 to rotate synchronously. The conductive sheet 704 is disposed to cooperate with the conductive ring 703, so that the outer sleeve 702 can rotate freely with the knob 9 while current conduction is ensured, without being limited by the conducting wires.

As shown in FIG. 2 and FIG. 4, a blade holder 301 of the blade 3 is inserted into the inner sleeve 701. The blade 3 is movably clamped in a movable slot 221 between the first jaw 201 and the second jaw 202. The movable slot 221 plays a limiting role on the telescopic direction of the blade 3.

As shown in FIG. 6 to FIG. 9, the blade control mechanism includes the triggering connecting rod 402, a blade feeding clip 403, and a blade reset spring 404. The blade feeding clip 403 is connected to the blade holder 301, and the blade holder 301 may rotate relative to the blade feeding clip 403. The blade reset spring 404 is sleeved on the inner sleeve 701, and abuts against the blade feeding clip 403. The control button 4 is connected to the blade feeding clip 403 through the triggering connecting rod 402. A button guide slot 405 is disposed inside the housing 1. The control button 4 slides along the button guide slot 405 and controls the blade feeding clip 403 to move along the inner sleeve 701, to drive the blade 3 to move. To improve flexibility of the connection between the triggering connecting rod 402 and the blade feeding clip 403, there is an oblong hole at a position at which the triggering connecting rod 402 is connected to the blade feeding clip 403.

As shown in FIG. 6 to FIG. 9, in the present invention, a convex post 8 is disposed on the control wrench 2, the convex post 8 is located above the locking lug boss 5. A concave portion matching the convex post 8 is disposed on the triggering connecting rod 402. The convex post 8 and the concave portion of the triggering connecting rod 402 form an auxiliary turnback mechanism. When the control wrench 2 is turned back, the convex post 8 abuts against the concave portion of the triggering connecting rod 402, and drives the triggering connecting rod 402 to synchronously turn back. The control button 4 synchronously turn back following the triggering connecting rod 402. The auxiliary turnback mechanism formed by the cooperation of the convex post 8 with the triggering connecting rod 402 may cooperate with the jaw reset spring 211 to have an auxiliary turnback function on the control button 4. When the jaw reset spring 211 pushes the control wrench 2 to turn back, the convex post 8 may drive the triggering connecting rod 402 to turn back synchronously, so as to drive the control button 4 to turn back synchronously.

In other feasible embodiments, the convex post 8 may not be disposed on the control wrench 2, and the locking lug boss 5 may be used to replace the function of the convex post 8, so that the locking lug boss 5 cooperates with the triggering connecting rod 402 to form an auxiliary turnback mechanism. A working principle is: When the control wrench 2 turns back, the locking lug boss 5 may abut against the triggering connecting rod 402 to drive the triggering connecting rod 402 to turn back synchronously, so as to drive the control button 4 to turn back synchronously.

It should be understood that although this specification is described in accordance with the embodiments, not each embodiment includes only one independent technical solution. The narrative mode in the specification is merely for clarity. A person skilled in the art should take the specification as a whole, and the technical solutions in all embodiments may also be appropriately combined to form other embodiments that may be understood by a person skilled in the art.

The series of detailed description listed above are merely specific description for the feasible embodiments of the present invention, and are not intended to limit the protection scope of the present invention. Any equivalent embodiment or modification made without departing from the technical spirit of the present invention should be included in the protection scope of the present invention.

## Claims

1. A safe high-frequency electrotome surgical instrument, comprising a housing (1), a shaft rod member, a jaw assembly, a control wrench (2), a blade (3), and a control button (4), wherein the control wrench (2) controls opening and closing of the jaw assembly through a jaw control mechanism; the control button (4) drives the blade (3) to move for cutting through a blade control mechanism; the moving direction of the control wrench (2) is consistent with that of the control button (4), and a locking mechanism is disposed between the control wrench (2) and the control button (4); the locking mechanism is composed of a locking lug boss (5) and an inverted buckle (6) capable of being clamped with the lug boss (5); the locking lug boss (5) is disposed on the control wrench (2); the inverted buckle (6) is disposed on the control button (4) or is disposed on a triggering connecting rod (402) of the blade control mechanism; in a first state, the jaw assembly is opened, and the locking lug boss (5) is clamped with the inverted buckle (6), such that the control button (4) is not movable; in a second state, the jaw assembly is closed, and the locking lug boss (5) is separated from the inverted buckle (6), such that the control button (4) is movable to drive the blade (3) for cutting; and after the control wrench (2) is turned back, the locking lug boss (5) is re-clamped with the inverted buckle (6).

2. The safe high-frequency electrotome surgical instrument according to claim 1, wherein the shaft rod member extends forward from the front end of the housing (1) and defines a longitudinal axis; the shaft rod member comprises an inner sleeve (701) and an outer sleeve (702); the jaw assembly is disposed at the front end of the shaft rod member; and the blade (3) is disposed inside the inner sleeve (701).

3. The safe high-frequency electrotome surgical instrument according to claim 2, wherein the jaw assembly comprises a first jaw (201) disposed at the front end of the inner sleeve (701) and a second jaw (202) disposed at the front end of the outer sleeve (702); the first jaw (201) is hingedly connected to the second jaw (202) through a first hinge pin (203); guide slots (204) are disposed on side portions of the tail ends of both the first jaw (201) and the second jaw (202); the front end of the inner sleeve (701) is connected to the first jaw (201) through a second hinge pin (205); the two ends of the second hinge pin (205) are movably clamped in the guide slots (204); the guide slot (204) is enclosed through a baffle (206); and the inner sleeve (701) drives the first jaw (201) and the second jaw (202) to be opened and closed by moving longitudinally.

4. The safe high-frequency electrotome surgical instrument according to claim 3, wherein the jaw control mechanism comprises a closing connecting rod (207), a push block (208), and a jaw spring (209); the push block (208) is sleeved on the inner sleeve (701), and abuts against the jaw spring (209) that is sleeved at the tail end of the inner sleeve (701); and the control wrench (2) is connected to the push block (208) through the closing connecting rod (207), and drives the push block (208) and the inner sleeve (701) to move longitudinally and synchronously.

5. The safe high-frequency electrotome surgical instrument according to claim 4, wherein a push-block guide slot (210) defining the moving direction of the push block (208) is disposed on the inner wall of the housing (1); and the push block (208) is slidably clamped in the push-block guide slot (210).

6. The safe high-frequency electrotome surgical instrument according to claim 5, wherein a jaw reset spring (211) abuts against the tail end of the push block (208); and the jaw reset spring (211) is clamped in a limiting slot (212) on the inner wall of the housing (1).

7. The safe high-frequency electrotome surgical instrument according to claim 1, wherein a wrench locking mechanism is disposed between the bottom portion of the control wrench (2) and the housing (1); the wrench locking mechanism comprises a locking block (213) located on the side portion of the control wrench (2) and a locking rod (214) located inside the housing (1); a clamping slot (215) containing a sliding guide surface is formed on the surface of the locking block (213); a limiting slot (218) is disposed inside the housing (1); the locking rod (214) moves elastically along the limiting slot (218), and the tail end of the locking rod (214) is pivotally connected to the housing (1); and there is a protruding portion (219) at the front end of the locking rod (214), so that the locking block (213) is enabled to drive, through movement relative to the locking rod (214), the protruding portion (219) to slide along the clamping slot (215).

8. The safe high-frequency electrotome surgical instrument according to claim 7, wherein a Z-shaped/concave guide surface (216) is formed on the surface of the clamping slot (215); an inward bending portion (220) is formed on the middle portion of the clamping slot (215); when the protruding portion (219) slides along the guide surface (216) to the inward bending portion (220), the locking block (213) and the locking rod (214) are locked; and when the protruding portion (219) slides out of the inward bending portion (220), the locking block (213) and the locking rod (214) are unlocked.

9. The safe high-frequency electrotome surgical instrument according to claim 8, wherein elastic members (217) abut against the upper and lower sides along the pivot direction of the locking rod (214) on the side wall of the housing (1).

10. The safe high-frequency electrotome surgical instrument according to claim 3, wherein two conductive rings (703) are respectively mounted outside the outer sleeve (702); and the two conductive rings (703) are electrically connected to the first jaw (201) and the second jaw (202) through conducting wires, respectively.

11. The safe high-frequency electrotome surgical instrument according to claim 10, wherein conductive sheets (704) slidably abut against the two conductive rings (703), respectively; the two conductive sheets (704) are fixedly disposed on the inner wall of the housing (1), and are connected to a power supply through conducting wires, respectively; a knob (9) is disposed at the front end of the housing (1); and the knob (9) is fixedly connected to the outer sleeve (702) and drives the outer sleeve (702) to rotate synchronously.

12. The safe high-frequency electrotome surgical instrument according to claim 4, wherein a blade holder (301) of the blade (3) is inserted into the inner sleeve (701); and the blade (3) is movably clamped in a movable slot (221) between the first jaw (201) and the second jaw (202).

13. The safe high-frequency electrotome surgical instrument according to claim 12, wherein the blade control mechanism further comprises a blade feeding clip (403) and a blade reset spring (404); the blade feeding clip (403) is connected to the blade holder (301); the blade reset spring (404) is sleeved on the inner sleeve (701), and abuts against the blade feeding clip (403); the control button (4) is connected to the blade feeding clip (403) through the triggering connecting rod (402); a button guide slot (405) is disposed inside the housing (1); and the control button (4) slides along the button guide slot (405) and controls the blade feeding clip (403) to move along the inner sleeve (701), to drive the blade (3) to move.

14. The safe high-frequency electrotome surgical instrument according to claim 1, wherein a convex post (8) is disposed on the control wrench (2), the convex post (8) is located above the locking lug boss (5); a concave portion matching the convex post (8) is disposed on the triggering connecting rod (402); the convex post (8) and the concave portion of the triggering connecting rod (402) form an auxiliary turnback mechanism; and when the control wrench (2) is turned back, the convex post (8) abuts against the concave portion of the triggering connecting rod (402), and drives the triggering connecting rod (402) to be synchronously turned back, wherein the control button (4) is synchronously turned back with the triggering connecting rod (402).

15. The safe high-frequency electrotome surgical instrument according to any one of claims 1 to 14, wherein the control wrench (2) is of a U-shaped open structure or an enclosed annular structure.
